# EUROPÄISCHE PATENTANMELDUNG

(11) **EP 2 359 894 A1**
(43) Veröffentlichungstag der Anmeldung: **24.08.2011**
(21) Anmeldenummer: 11153005.1
(22) Anmeldetag: 02.02.2011
(51) Int. Cl.: A61N 1/05, A61N 1/08

(54) **Elektrode mit HF-Filter für aktive medizinische Implantate**

(30) Priorität: 11.02.2010 DE 102010000367; 11.02.2010 DE 102010000369; 11.02.2010 DE 102010000370; 11.02.2010 DE 102010000371; 11.02.2010 DE 102010000372
(71) Anmelder: BIOTRONIK SE & Co. KG, 12359 Berlin (DE)
(72) Erfinder: Täubert, Kerstin, 12589, Berlin (DE); Lenski, Hartmut, 15806, Glienicke (DE); Weiss, Ingo, 12435, Berlin (DE); Friedrich, Michael, 14532, Kleinmachnow (DE); Knorr, Stefan, 10119, Berlin (DE); Fischer, René, 10247, Berlin (DE); Schurr, Marc Steffen, 10179, Berlin (DE)
(74) Vertreter: Lindner-Vogt, Karin L.

(57) **Zusammenfassung**

Eine Elektrodenvorrichtung für aktive medizinische Implantate umfasst
- einen langgestreckten Elektrodenkörper (2) mit einem proximalen und einem distalen Ende (1),
- einen Spitzen-Kontaktpol (6) am distalen Ende (1),
- mindestens einen Ring-Kontaktpol (5) vor dem distalen Ende (1),
- elektrischen Zuleitungen (3, 4) zu dem Spitzen- und Ring-Kontaktpol (6, 5), und
- einen Hochfrequenz-Filter (11) vor dem distalen Ende (1), der elektrischen Kontakt zum Spitzen-Kontaktpol (6) aufweist und in dessen Zuleitung (4) eingeschaltet ist.

## Beschreibung

Die Erfindung betrifft eine Elektrodenvorrichtung für aktive medizinische Implantate.

Zum Hindergrund der Erfindung ist festzuhalten, dass der Erfindungsgegenstand in erster Linie im Zusammenhang mit Herzschrittmachern, implantierbaren Defibrillatoren und anderen Typen von aktiven implantierbaren elektromedizinischen Geräten relevant ist. Letztere weisen in aller Regel mindestens eine strom-/spannungsführende Zuleitung in der Elektrodenvorrichtung - üblicherweise als kurz als "Elektrode" bezeichnet - auf, deren distales Ende beispielsweise in einer Herzkammer angeordnet und zur Messung kardiologischer Potentialsignale oder zur Abgabe entsprechender therapeutischer Stromsignale dient.

Die Kompatibilität derartiger Elektrodenvorrichtungen bei implantierbaren elektromedizinischen Geräten mit hochfrequenten Magnetfeldern, wie sie insbesondere bei bildgebenden diagnostischen Verfahren auf Magnetresonanz-Basis - sogenannte MRI- (magnetic resonance imaging) Verfahren ― verwendet werden, stellt ein gravierendes Problem dar. Bei solchen MRI -Verfahren wird einem starken statischen Magnetfeld ein mit Radiofrequenz (RF) gepulstes magnetisches Wechselfeld überlagert, das dazu dient, den Energiestatus der Protonen im untersuchten Gewebe zu verändern und entsprechende MRI-Signale aus dem Gewebe zu produzieren.

Dieses magnetische Wechselfeld führt nach den Gesetzen der elektromagnetischen Induktion in den Zuleitung der hier in Rede stehenden Elektrodenvorrichtungen von elektromedizinischen Geräteimplantaten zu Wechselspannungen, deren Energie insbesondere an den elektrisch leitenden Kontaktpolen der Elektrodenvorrichtung zum menschlichen Gewebe in Wärme umgesetzt wird. Dies kann zu einer erheblichen Erhitzung beispielsweise des Spitzenkontaktes einer Herzelektrode mit einer entsprechenden Beeinträchtigung und sogar Schädigung des damit in Berührung stehenden oder umliegenden Herzgewebes führen.

Um diese Problematik zu vermeiden, schlägt die US 7,363,090 B2 die Verwendung von Filtern auf der Basis von Schwingkreisen aus parallel geschalteter Spule und Kondensator vor, die der entsprechenden Zuleitung für den Spitzen-Kontaktpol oder einen Ring-Kontaktpol einer entsprechenden Elektrode eines implantierbaren elektromedizinischen Gerätes zugeordnet ist. Die in diesem vorbekannten Patent offenbarten Filter sind in der Praxisumsetzung der Patentinhaberin als vergleichsweise voluminöse, die Elektrodenvorrichtung auf einer gewissen Länge versteifende Komponenten ausgebildet, die der damit ausgerüsteten Elektrode ungünstige mechanische Eigenschaften verleihen. Ferner ist der Filter in einem in sich geschlossenen Gehäuse untergebracht, das keine Durchführung für die in aller Regel beim Implantieren einer Elektrode verwendeten Führungsdrähte bereit stellt. Insoweit sind die Einsatzmöglichkeiten dieser bekannten Elektrode mit Filtereinrichtung begrenzt.

Aus der US 2009/0281592 A1 sind Filterkomponenten zur Herabsetzung der Erhitzung von Schrittmacher-Elektroden eines elektromedizinischen Implantats bei der Einwirkung hochfrequenter Magnetfelder in Folge von MRI-Prozeduren bekannt, wobei eine Induktionsspule um einen nicht-leitenden, zentralen Abschnitt eines Schaftes aufgebracht ist, der einen Spitzen-Kontaktpol mit einem inneren Wendelleiter der Elektrodenvorrichtung verbindet. Durch die Montage einer Induktionsspule auf dem Schaft wird eine induktive Signalfilterung zur Herabsetzung der Erhitzung der Elektrodenspitze ohne die Notwendigkeit eines vergleichsweise langen, voluminösen Spulenkörpers entlang der Länge der Elektrode erreicht. Kapazitive Elemente können ebenfalls in den Schaft integriert werden, um einen LC-Filterkreis zu schaffen. Alternativ dazu ist in dieser Druckschrift eine sogenannte "Luftspule" als induktives Element offenbart, bei der der Schaft weggelassen werden kann.

Die Filtereinrichtungen nach dem Stand der Technik führen in der Regel zu einer übermäßigen Versteifung der Elektrodenvorrichtung auf einer bestimmten Länge.

Davon ausgehend liegt der Erfindung die Aufgabe zugrunde, Elektrodenvorrichtungen für aktive medizinische Implantate so zu verbessern, dass eine möglichst große Flexibilität des Elektrodenkörpers durch vorteilhafte Einbaupositionen des Hochfrequenz-Filters erzielt wird.

Diese Aufgabe wird durch die im Patentanspruch 1 angegeben Merkmale einer Elektrodenvorrichtung gelöst, die umfasst:
- einen langgestreckten Elektrodenkörper mit einem proximalen und einem distalen Ende,
- einen Spitzen-Kontaktpol am distalen Ende,
- mindestens einen Ring-Kontaktpol vor dem distalen Ende,
- elektrischen Zuleitungen zu dem Spitzen- und Ring-Kontaktpol, und
- einen Hochfrequenz-Filter vor dem distalen Ende, der elektrischen Kontakt zum Spitzen-Kontaktpol aufweist und in dessen Zuleitung eingeschaltet ist.

Aufgrund dieser erfindungsgemäß konzeptionellen Ausbildung einer Elektrodenvorrichtung können je nach Zielsetzung bei der jeweiligen Elektrodenvorrichtung verschiedene Spezifikationen bezüglich der Flexibilität der Elektrodenvorrichtung realisiert werden. So ist durch eine zumindest teilweise radial überlappende Positionierung von Hochfrequenz-filter und ―kontaktpol zueinander eine mehr oder weniger enge Beabstandung von Spitzen- und Ring-Kontaktpol unter gleichzeitiger Einbeziehung des Hochfrequenz-Filters möglich, so dass ein kurzer Polabstand zwischen Spitze und Ring bei gleichzeitiger Kompaktheit erzielt werden kann.

Vorteilhafterweise kann bei einer solchen teilweisen Überlappung die wendelförmige Zuleitung für den Ring-Kontaktpol in proximaler Richtung vor dem Hochfrequenz-Filter enden. In axialer Richtung sind also Hochfrequenz-Filter und ―zuleitung axial entzerrt, so dass ein insgesamt dünnerer Elektrodenkörper geschaffen werden kann. Zwischen dem Hochfrequenz-Filter und der Ringelektrode muss dann nämlich nicht mehr die Zuleitung selbst untergebracht werden.

Eine schaltungstechnisch rationelle Anbindung von Ring-Kontaktpol und Hochfrequenz-Filter ist gegeben, wenn die äußere Zuleitung für den Ring-Kontaktpol gleichzeitig mit der äußeren Gehäusefläche des Hochfrequenz-Filters in elektrischem Kontakt steht. Diese Gehäusefläche ist dann wiederum an die entsprechenden Filterkomponenten im Gehäuseinneren elektrisch angekoppelt.

Eine weitere Minimierung des Durchmessers des Elektrodenkörpers ist bei einer bevorzugten Ausgestaltung erzielbar, bei der der Ring-Kontaktpol in proximaler Richtung derart vor dem Hochfrequenz-Filter angeordnet ist, dass sich Ring-Kontaktpol und Hochfrequenz-Filter außerhalb axialer Überdeckung befinden. Dabei ist der Innendurchmesser des Ring-Kontaktpols gegebenenfalls mit seiner wendelförmigen Zuleitung kleiner als der Außendurchmesser des Hochfrequenz-Filters. Bei dieser Ausführungsform sind also Filter und Ringpol in axialer Richtung hinsichtlich ihrer Positionierung vollständig getrennt, so dass im Extremfall der Außendurchmesser des Hochfrequenz-Filters gegebenenfalls mit einer Isolierung dem Außendurchmesser der Ringelektrode entsprechen kann.

Die Flexibilität der Elektrodenvorrichtung im Bereich der Spitze wird durch eine weitere bevorzugte Ausführungsform verbessert, indem der Hochfrequenz-Filter mit dem Spitzen-Kontaktpol mechanisch und elektrisch durch ein flexibles Wendelstück verbunden ist. Das Wendelstück wirkt damit im Sinne einer Doppelfunktion als elektrische Zuleitung und als mechanisches Gelenk, so dass bei einer entsprechend flexiblen, isolierenden Ausgestaltung des Elektrodenkörpers in diesem Bereich durch die Anbindung des Spitzen-Kontaktpols an den Hochfrequenz-Filter keine nennenswerte Flexibilitätseinbußen zu verzeichnen sind.

Gemäß weiteren bevorzugten Varianten der Erfindung kann der Spitzen-Kontaktpol ein Kopf mit elektrisch aktiver Fixierschraube, ein Linsen-, Kegel- oder halbkugelförmiger Kopf, ein verlängerter, als Fixierschraube ausgebildeter Kontaktpin des Hochfrequenz-Filters oder ein Fortsatz des Gehäuses des Hochfrequenz-Filters selbst sein. Erkennbar eröffnet die Erfindung also ausgesprochen variable Realisierungsmöglichkeiten für den Spitzen-Kontaktpol.

Eine Weiterbildung des Erfindungsgegenstandes zielt ab auf eine Einbeziehung des Hochfrequenz-Filters in den mechanischen Drehantrieb eines als elektrisch aktive Fixierschraube ausgebildeten Spitzen-Kontaktpols. Demnach ist der Hochfrequenz-Filter gemeinsam mit dem Spitzen-Kontaktpol in der Elektrodenvorrichtung, insbesondere innerhalb des Ring-Kontaktpols um die Längsachse der Elektrodenvorrichtung drehbar gelagert. Vorzugsweise werden dazu Lagerscheiben mit Lagerbohrungen in der Elektrodenvorrichtung bereitgestellt, in denen der Hochfrequenz-Filter mit seinen Kontaktpins drehbar gelagert und durch die elektrische Zuleitung des Spitzen-Kontaktpols rotationsangetrieben ist. Damit lässt sich eine leichtgängige Drehbarkeit und damit für den Operateur gut handhabbare Fixierung des Spitzen-Kontaktpols der Elektrodenvorrichtung am Diagnose-/Therapieort erreichen.

Gemäß einer weiteren bevorzugten Ausführungsform weist der Hochfrequenz-Filter elektrische Kontaktpins mit dazwischen geschalteten elektronischen Miniatur-Bauelementen auf, wobei Kontaktpins und Miniatur-Bauelemente gemeinsam stoffschlüssig in einem umspritzten Filtergehäuse angeordnet sind. Durch diese Filterausgestaltung ist ein kompakter, mechanisch robuster und elektrisch sauber isolierter Filteraufbau erreichbar, der für den Einbau in der Elektrodenvorrichtung besonders geeignet ist.

Durch eine funktionale Beschichtung, wie beispielsweise in Form einer Dampfsperre, einer Keramik- oder Metallbeschichtung kann das Filtergehäuse an die entsprechenden Einsatzbedingungen optimal angepasst werden.

Diesem Zweck dient auch die weitere Fortbildung der erfindungsgemäßen Elektrodenvorrichtung, wonach die Kontaktpins jeweils als Röhrchen ausgebildet sind, deren Lumen mit einem Durchlass im Filtergehäuse als Durchgang für einen Führungsdraht zur Implantierung der Elektrodenvorrichtung fluchten. Damit ist die Einführbarkeit der Elektrodenvorrichtung durch die Verwendbarkeit eines Führungsdrahtes trotz des integrierten Hochfrequenz-Filters verbessert.

Eine konzeptionelle Weiterbildung der Erfindung bezieht sich wieder auf die Gehäusegestaltung des Hochfrequenz-Filters, dessen Kontaktpins als mit ihren Rändern einander zugewandte, voneinander isolierte Kontaktkappen ausgebildet sein können. Damit wird eine Art Tonnenfilter geschaffen, in dessen Innenraum die elektrischen Filterkomponenten untergebracht sind. Vorteilhafte elektrische Anbindungen dieser Komponenten an die Kontaktkappen sehen Schleifkontakte oder Kontaktfedern zwischen den Anschlüssen der elektrischen Komponenten und der Innenseite der Kontaktkappen vor.

Schließlich ist es möglich, den Hochfrequenz-Filter direkt in den Spitzen-Kontaktpol zu integrieren, was ebenfalls eine Verringerung der Flexibilität des Elektrodenkörpers verhindert.

Außerdem ist es möglich die aufgeführte Technik für Katheter, insbesondere für Ablationskatheter, zu nutzen.

Weitere Merkmale, Einzelheiten und Vorteil der Erfindung ergeben sich aus der nachfolgenden Beschreibung von Ausführungsbeispielen anhand der beigefügten Zeichnungen. Es zeigen:
- Fig. 1 bis 5: koaxiale Längsschnitte einer Elektrodenvorrichtung in deren distalem Endbereich in unterschiedlichen Ausführungsformen,
- Fig. 6 bis 11: höchst schematische Schnittdarstellungen unterschiedlicher Filter-Spitzen-Kontaktpol-Konfigurationen,
- Fig. 12 bis 19: höchst schematische Darstellungen von Hochfrequenz-Filtern in verschiedenen Ausführungsformen,
- Fig. 20 und 21: Teildarstellungen von Hochfrequenz-Filtern, und
- Fig. 22 bis 31: schematische koaxiale Längsschnitte von Spitzen-Kontaktpolen mit integrierten Hochfrequenz-Filtern in unterschiedlichen Ausführungsformen.

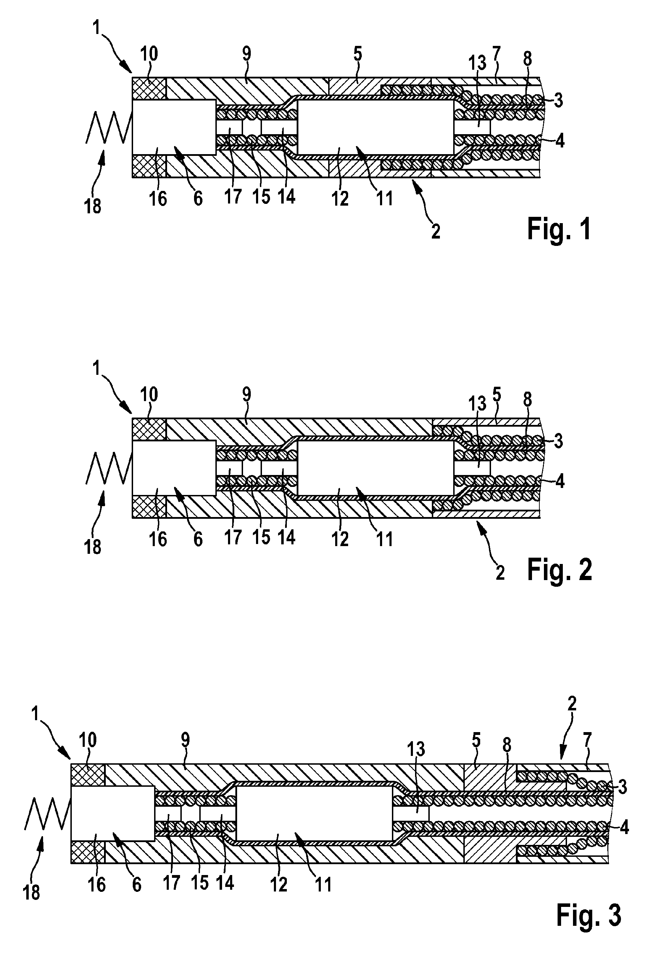

Anhand von Fig. 1 soll der grundsätzliche Aufbau einer Elektroden-Vorrichtung, wie sie beispielsweise als Schrittmacher-Elektrode eines implantierbaren Herzschrittmachers zum Einsatz kommt, erläutert werden. Sofern die Beschreibungen der weiteren Ausführungsbeispiele gemäß den Fig. 2 bis 31 keine gegenteiligen Ausführungen erkennen lassen, gelten die entsprechenden Erörterungen auch für diese Ausführungsformen, wobei identische Bezugszeichen übereinstimmende Bauteile bezeichnen und damit nicht nochmals näher erörtert werden müssen.

In Fig. 1 ist der Bereich vor dem distalen Ende 1 der Elektrodenvorrichtung gezeigt, mit dem ein langgestreckter Elektrodenkörper 2 abgeschlossen ist. Darin verlaufen zwei wendelförmige Zuleitungen 3, 4, nämlich eine äußere Zuleitung 3 zu einer vor dem distalen Ende 1 angeordneten Ringelektrode 5 und eine innere Zuleitung 4, die für den elektrischen Anschluss des Spitzen-Kontaktpols 6 am distalen Ende 1 der Elektrodenvorrichtung in noch näher zu erläuternder Weise sorgt.

Der Elektrodenkörper 2 ist außen durch einen isolierenden Schlauch 7 abgeschlossen. Zwischen den Zuleitungen 3, 4 befindet sich eine ebenfalls schlauchförmige Isolationslage 8. Zwischen Ring-Kontaktpol 5 und Spitzen-Kontaktpol 6 ist eine flexible Isolationshülse 9 gesetzt, die durch einen Steroidkragen 10 abgeschlossen ist.

Den Ausführungsformen gemäß den Fig. 1 bis 5 ist gemein, dass ein Hochfrequenz-Filter 11 im Bereich vor dem distalen Ende 1 der Elektrodenvorrichtung eingebaut ist. Dieser Filter, der beispielsweise aus einer Spule mit einer Induktivität L und einem Kondensator mit einer Kapazität C als LC-Schwingkreis aufgebaut ist, dient zur Ausfilterung der eingangs erwähnten hochfrequenten Ströme, die durch das entsprechende Magnetfeld bei MRI-Systemen induziert wird. Damit wird einer Erhitzung des Ring- und insbesondere Spitzen-Kontaktpols 5, 6 wirkungsvoll vorgebeugt. Bei den Ausführungsformen gemäß Fig. 1 bis 5 ist der Hochfrequenz-Filter 11 als einheitliches Bauteil mit einem zylinderförmigen Gehäuse 12 und koaxial zur proximalen bzw. distalen Seite hin an den Gehäusestirnseiten angeordneten Anschlüssen 13, 14 gezeigt.

Bei der Ausführungsform gemäß Fig. 1 sitzt der Ring-Kontaktpol 5 etwa mittig und damit vollständig in axialer Richtung überlappend auf den Hochfrequenz-Filter, wobei zur elektrischen Trennung die Isolationslage 8 zwischen Ring-Kontaktpol 5 und Filter 11 hindurchgezogen ist. Auf die Isolationslage 8 läuft das Ende der äußeren wendelförmigen Zuleitung 3 auf und ist mit dem Ring-Kontaktpol 5 elektrisch verbunden.

Zum distalen Ende hin ist auf den Anschluss 14 ein flexibles Wendelstück 15 aufgesetzt, das den Kopf 16 des Spitzen-Kontaktpols 6 mechanisch flexibel anbindet und elektrisch über dessen Anschluss 17 kontaktiert. Das Wendelstück 15 wirkt damit als Gelenk, so dass der gesamte Endbereich am distalen Ende 1 sich gut an Biegungen in dem die Elektroden-vorrichtung aufnehmenden Gefäß, insbesondere beim Einführen anpasst.

Der Spitzen-Kontaktpol 6 ist im gezeigten Ausführungsbeispiel gemäß Fig. 1 als Fixierhelix 18 ausgelegt, deren korkenzieherartiges Schraubteil zum Fixieren der Elektrodenvorrichtung im Körpergewebe in bekannter Weise dient.

Alternativ zu der gezeigten Fixierhelix können in üblicher Weise Silikonanker vorgesehen sein.

Wie in den Zeichnungen nicht gesondert dargestellt ist, können Elektrodenvorrichtungen mit einem integrierten Filter auch einen Ring-Kontaktpol 5 besitzen, der nicht als geschlossener Ring ausgeführt ist. Stattdessen besteht der elektrische Kontakt des Ring-Kontaktpols aus einer Struktur, die beispielsweise aus einem oder mehreren Ringsegmenten besteht. Vorteil einer solchen Lösung ist der zusätzliche Platz im Querschnitt der Elektrode, um beispielsweise elektrische Komponenten besser integrieren zu können.

Die in Fig. 2 gezeigte Variante der Elektrodenvorrichtung unterscheidet sich von der Ausführungsform gemäß Fig. 1 in der Positionierung des Ring-Kontaktpols 5, der in proximaler Richtung nach hinten versetzt ist und folglich mit dem Hochfrequenz-Filter 11 lediglich teilweise überlappt. Dabei endet die äußere Zuleitung 3 für den Ring-Kontaktpol 5 proximal vor dem Hochfrequenz-Filter 11, so dass kein Bauraum zwischen Ring-Kontaktpol 5 und Filter 11 für diese Zuleitung 3 zur Verfügung gestellt werden muss. Insoweit kann die gesamte Elektrodenvorrichtung also im Durchmesser kleiner ausgeführt werden.

Dieser Effekt wird bei der Ausführungsform gemäß Fig. 3 noch verstärkt, da hier Ring-Kontaktpol 5 und Hochfrequenz-Filter 11 völlig außer längsaxialer Überdeckung stehen. Vielmehr ist der Ring-Kontaktpol 5 in proximaler Richtung vor dem Hochfrequenz-Filter 11 angeordnet und weist einen Innendurchmesser d auf der, der kleiner als der Außendurchmesser D des Filters 11 ist. Insoweit kann die Ring-Elektrode und damit die gesamte Elektrodenvorrichtung in ihrem Außendurchmesser noch kleiner bemessen werden. Die Isolationshülse 9 ist in den Ausführungsformen gemäß Fig. 2 und 3 teilweise oder komplett über die Isolationslage 8 und den Hochfrequenzfilter 11 bis zum Ring-Kontaktpol 5 fortgesetzt.

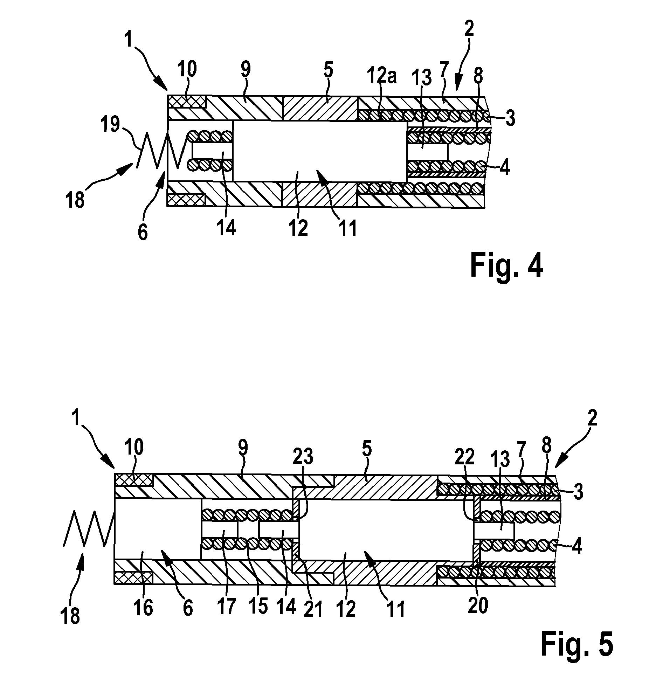

Bei der in Fig. 4 gezeigten Ausführungsform ist der Ring-Kontaktpol 5 wieder mittig auf den Hochfrequenz-Filter 11 gesetzt, wobei hier die Isolationslage 8 weg gelassen ist. Die Kontaktierung der elektrischen Komponenten des Hochfrequenz-Filters 11 erfolgt dabei über die Außenfläche 12a des Gehäuses 12, die zusammen mit dem Ring-Kontaktpol 5 an die äußere Zuleitung 3 angeschlossen und damit elektrisch kontaktiert ist.

Ein weiterer Unterschied zu den Ausführungsformen gemäß Fig. 1 bis 3 besteht in der Ausführung der Fixierhelix 18. Deren Schraubteil 19 ist direkt auf den distalen Anschluss des Hochfrequenz-Filters 11 aufgesetzt. Das Einschrauben der Fixierhelix 18 erfolgt dabei - in Übereinstimmung mit den Varianten nach Fig. 1 bis 3 ― durch eine Drehung des gesamten Elektrodenkörpers 2 um seine Längsachse.

In der Ausführungsform gemäß Fig. 5 wird ein davon abweichendes Konzept für den Drehantrieb der Fixierhelix 18 realisiert, indem der Hochfrequenz-Filter 11 in quer zur Längsachse des Elektrodenkörpers 2 angeordneten Lagerscheiben 20, 21 drehbar gelagert ist, in dem seine Kontaktpins 13, 14 in entsprechenden Lagerbohrungen 22, 23 der Lagerscheiben 20, 21 sitzen. Am proximalen Kontaktpin 13 ist die innere Zuleitung 4 mechanisch befestigt und elektrisch angeschlossen. Das Wendelstück 15 verbindet den distalen Kontaktpin 14 mit dem Anschluss 17 des Kopfes 16 der Fixierhelix 18, die hier in der Isolationshülse 9 drehbar gelagert ist. Insgesamt erfolgt die Betätigung der Fixierhelix 18 durch eine Drehung des (nicht gezeigten) Steckerpins am proximalen Ende der Elektrodenvorrichtung. Diese Drehung wird über die innere Zuleitung 4 auf den Hochfrequenz-Filter 11 und weiter über das Wendelstück 15 bis zur Fixierhelix 18 übertragen.

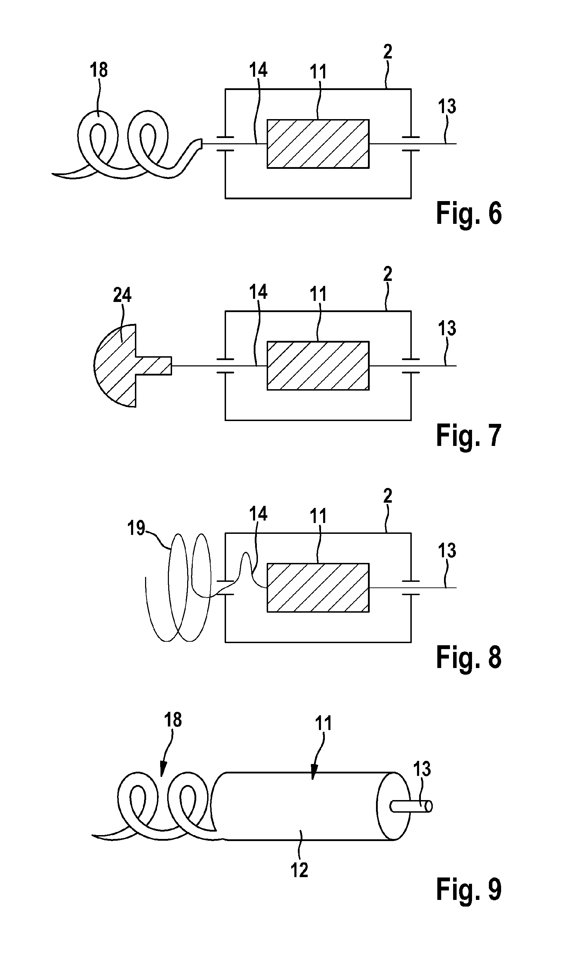

Die Fig. 6 bis 8 zeigen überblickartig verschiedene Konzepte für die Ausführungen und Anbindung von Spitzen-Kontaktpolen 6 an den Hochfrequenz-Filter 11. Generalistisch ist in diesen Figuren der Elektrodenkörper 2 ohne detaillierte Darstellung seines Aufbaus am distalen Ende 1 als schlichter Kasten dargestellt.

Fig. 6 gibt im Wesentlichen die in Fig. 1 bis 5 detaillierter dargestellte Konfiguration wieder, bei der der distale Kontaktpin 14 des Filters 11 mit einer Fixierhelix 18 verbunden ist.

Bei der in Fig. 7 dargestellten Ausführungsform wird ein halbkugelförmiger Kopf 24 verwendet, der an dem Kontaktpin 14 des Filters angeordnet ist.

Bei der Variante gemäß Fig. 8 ist das Schraubteil 19 direkt aus dem distalen Kontaktpin 14 des Filters gebildet und aus dem Elektrodenkörper 2 herausgeführt.

Fig. 9 zeigt einen Hochfrequenz-Filter 11, dessen Gehäuse 12 eine direkt angeformte Fixierhelix 18 besitzt, die dann auch entsprechend elektrisch an die Komponenten des Filters 11 angebunden ist.

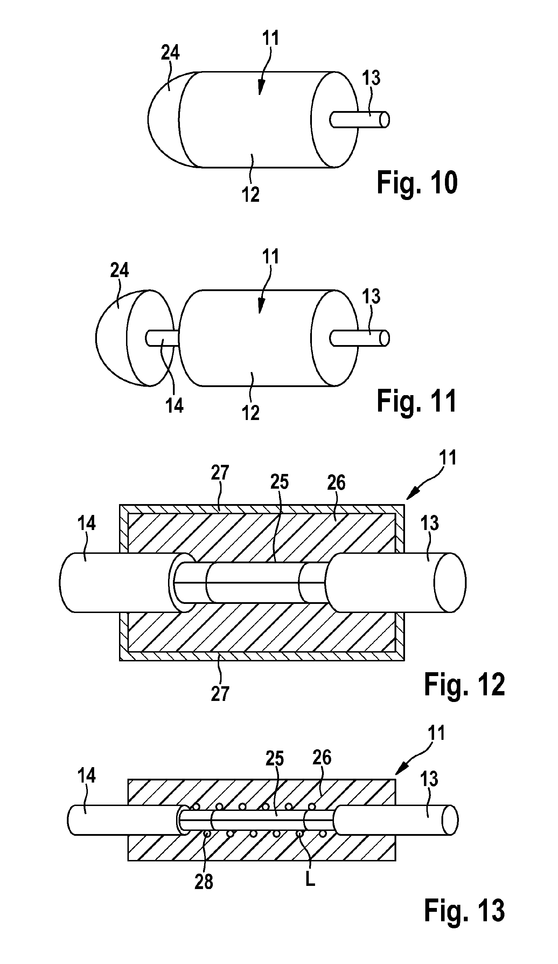

Bei der Ausführungsform gemäß Fig. 10 besitzt das Gehäuse 12 des Filters 11 einen direkt angeformten halbkugelförmigen Kopf 24, der Teil des Gehäuses ist oder daran anliegt. Bei Fig. 11 ist eine räumliche Trennung zwischen diesem halbkugelförmigen Kopf 24 über den Kontaktpin 14 gezeigt.

Die halbkugelförmigen Köpfe 24, wie sie in Fig. 7, 10 und 11 gezeigt sind, können auch linsen-, kegel- oder anders geformt sein.

Die im Zusammenhang mit Fig. 6 bis 11 gezeigten Ausführungsformen des Filters 11 und seiner Kombination mit Spitzen-Kontakt-Polen 6 dienen dazu, dass der Filter besser in die Elektrodenvorrichtung eingebaut werden und zusätzliche Funktionen übernehmen kann. Die Integrierbarkeit des Filters wird dabei gegenüber herkömmlichen Filterdesigns erhöht. Die Elektrodenvorrichtung lässt sich so gestalten, dass sie weniger physiologische Probleme verursacht, da sie flexibler, dünner und mit kürzeren steifen Bereichen ausgelegt werden kann. Dadurch reduziert sich die Gefahr von beispielsweise Perforationen des Herzmuskels erheblich.

Die Kontaktpole 5, 6 in jedweder Ausführung, die entsprechenden Gehäuse(-abschnitte) oder Teile davon können mit einer beispielsweise fraktalen Beschichtung versehen sein. Die restlichen Gehäusebereiche können alternativ oder zusätzlich mit einer nicht leitenden Beschichtung überzogen werden. Diese kann aus Silikon, Keramik, einer anorganischen Schicht, einer DLC- (diamand like carbon-) Schicht, einem Kunststoff, wie Polyurethan, thermoplastischen Polyurethan, Parylene usw. bestehen. Das Gehäuse der Filter 11 selbst kann aus einem Metall oder einer Metalllegierung gefertigt sein, vorzugsweise aus Edelstahl, aus Platin, Titan oder einer Platin/Iridiumlegierung. Es ist ebenso möglich, das Gehäuse aus einer Keramik oder einem Kunststoff zu fertigen, wobei diese Materialien mit einem leitfähigen Material überzogen oder selbst leitfähig sein können. Für manche Filtertypen ist es vorteilhaft einen zusätzlichen Kontakt (so was wie einen Massebezug oder ein Referenzpotential etc.) zum Gewebe zu haben. Das elektrisch leitende Gehäuse kann diesen Kontakt herstellen. Für andere Filtertypen ist so ein Kontakt unerwünscht, jedoch ggf. eine metallisch leitende Abschirmung (oder auch nur wegen mechanischer Festigkeit, wegen Wärmeableitung etc) vorteilhaft. Daher in solchen Fällen das nichtleitfähige/nichtleitfähig beschichtete Gehäuse zu bevorzugen.
In anderen Fällen ist elektrischer Kontakt gewünscht, allerdings nicht bei allen Frequenzen, vor allen nicht bei Gleichstrom. Daher dient die isolierende Schicht als Dielektrikum zwischen dem Metallischen Gehäuse und dem Gewebe. Zusammen bilden diese einen Kondensator.

In den Fig. 12 bis 15 sind alternative Aufbaukonzepte für den Hochfrequenz-Filter 11 dargestellt, wie sie ebenfalls zum Einbau in Elektroden für einen Herzschrittmacher, Defibrillator, Neurostimulator oder ähnliche aktive medizinische Implantaten geeignet ist. Gehäuse von vorbekannten Filtern sind in der Regel aus massiven Metallteilen aufgebaut, wobei für den Aufbau einer Isolation zwischen Gehäuse und elektrischen Bauelementen meist teure keramische Bauteile verwendet werden. Die Abdichtung des Gehäuses ist sehr aufwändig, problematisch und damit kostenintensiv. Durch die in den Fig. 12 bis 15 gezeigten Konzepte ist es möglich, eine konstruktiv einfache Abdichtung gegen Flüssigkeiten zu erreichen, so dass der Hochfrequenz-Filter 11 kostengünstig zu realisieren ist. Verschiedenste elektrische Baukomponenten können dabei einfach und variabel eingebettet werden, da das Gehäuse im Wesentlichen durch eine Umspritzung und gegebenenfalls diversen Vor- und Nachbehandlungsschritten entsteht.

Im Detail zeigt Fig. 12 die beiden kollinear mit Abstand angeordneten Kontaktpins 13, 14 des Filters 11, zwischen die eine oder mehrere elektrische Bauteile 25 in entsprechender Verschaltung eingebracht und damit verbunden sind. Unter Freilassung der Enden der Kontaktpins 13, 14 wird diese gesamte Baugruppe mit einem Kunststoffkörper 26 umspritzt, der die Abdichtung und elektrische Isolierung der Bauteile 25 gewährleistet.

Bei Bedarf kann der so entstandene Filter 11 noch mit einer Beschichtung 27 versehen werden, die beispielsweise aus einem Kunststoff, einer Keramik oder einer anderweitigen anorganischen Schicht bestehen kann. Eine solche funktionale Beschichtung 27 dient dazu, die Oberflächeneigenschaften an entsprechende Einsatzbedingungen anzupassen, beispielsweise kann die Beschichtung 27 eine mechanische Stabilisierung oder die Ausbildung einer Dampfsperre mit sich bringen.

Die in Fig. 13 dargestellte Ausführungsform unterscheidet sich von der in Fig. 12 dadurch, dass die elektrischen Bauteile 25 zwischen den Kontaktpins 13, 14 noch mit einer Drahtspule 28 umwickelt sind, die die Induktivität L des Hochfrequenz-Filters realisieren kann.

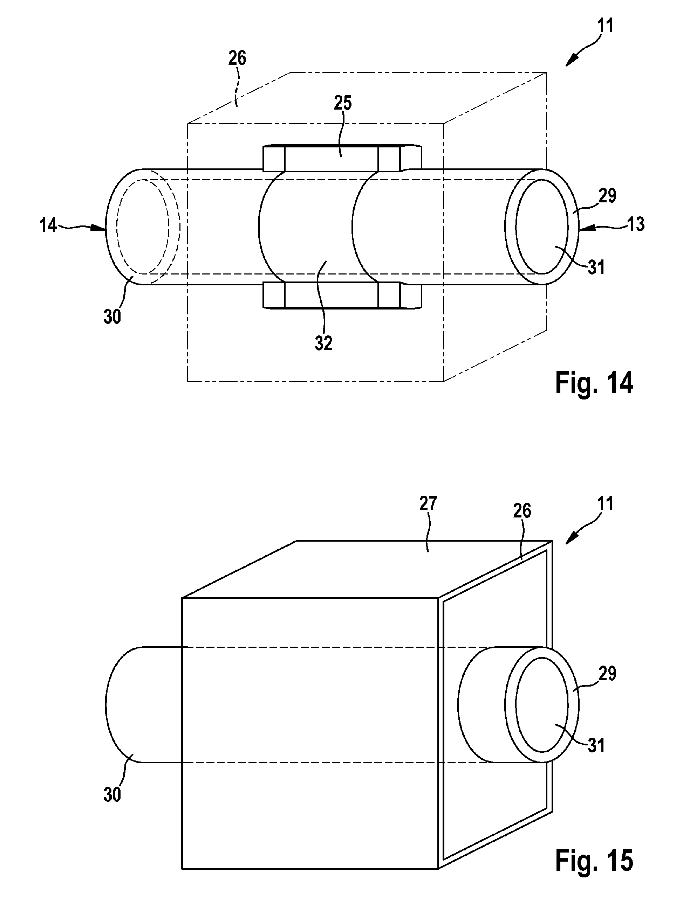

Um eine Elektrodenvorrichtung mit einem Hochfrequenz-Filter 11 zu versehen und gleichzeitig die Verwendung eines Führungsdrahtes zu ermöglichen, zeigt Fig. 14 eine Ausführungsform, bei der die Kontaktpins 13, 14 als leitfähige Röhrchen 29, 30 ausgebildet sind, deren Lumen 31 mit einem entsprechenden Durchlass 32 im das Filtergehäuse 12 bildenden Kunststoffkörper 26 fluchtet. Durch Lumen 31 und Durchlass 32 kann dann ein Führungsdraht, Guidewire, Mandrin o. ä. passieren. Wie aus Fig. 14 deutlich wird, sind dabei die elektrischen Bauteile 25 seitlich gegenüber dem Durchlass 32 versetzt eingebettet.

Fig. 15 zeigt nochmals eine Außenansicht des Filters gemäß Fig. 14, wobei auf das Gehäuse wieder eine Beschichtung 27 aus Metall, verschiedenen Kunststoffen bzw. anorganischen oder organischen Verbindungen entsprechend der gewünschten Funktionalität aufgebracht ist.

Die elektrischen Kontaktpins 13, 14 bzw. Röhrchen 29, 30 können aus Edelstahl, Platin, Platin/Iridium-Legierung oder Titan realisiert werden. Auch können sie mit einzelnen oder mehreren Bohrungen, Rillen, Gravierungen oder Vertiefungen versehen sein, um die mechanische Festigkeit des Filters 11 nach der Umspritzung zu erhöhen und diesen insgesamt zu stabilisieren.

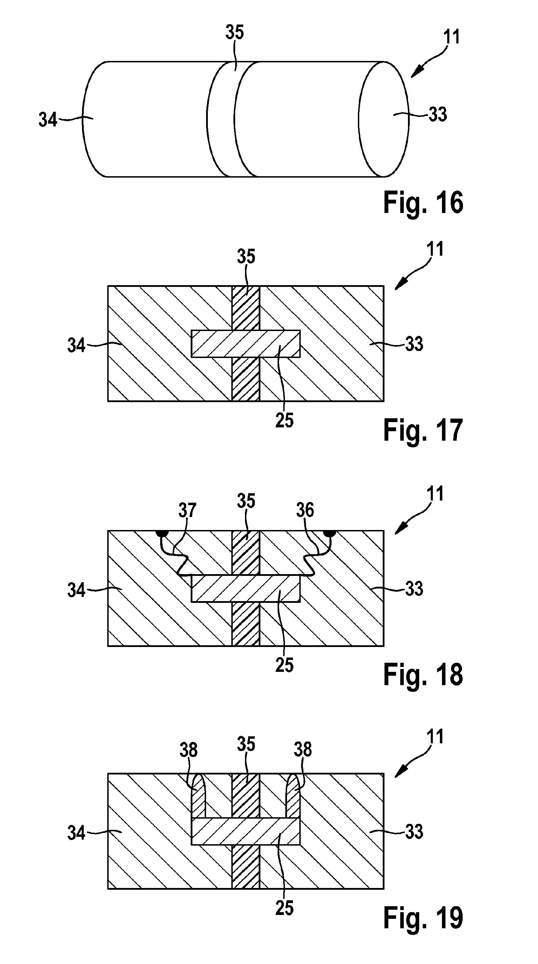

Die folgenden Fig. 16 bis 21 zeigen Ausführungsformen eines Hochfrequenz-Filters 11, die keine Kontaktpins 13, 14 mehr benötigen und deren Gehäuse damit auf einfache Art und Weise abgedichtet werden kann. Bei den vorher beschriebenen Varianten des Filters 11 vergrößern die Kontaktpins 13, 14 die Baugröße des Filters 11, es müssen zusätzliche Passagen isoliert oder abgedichtet werden.

Wie aus der Ansicht gemäß Fig. 16 und dem schematischen Schnitt gemäß Fig. 17 deutlich wird, sind die Kontaktpins durch zwei voneinander isolierte Kontaktkappen 33, 34 gebildet, die über einen Isolatoreinsatz 35 mechanisch verbunden und elektrisch isoliert werden. Die beiden durch die Kontaktkappen 33, 34 gebildeten "Halbtonnen" sind wasserdicht verbunden, es entstehen zwei elektrisch getrennte Bereiche.

In dem Isolatoreinsatz 35 sitzen die elektrischen Bauteile 25 in entsprechender Konfiguration, so dass sie beispielsweise ein Hochpass-, Tiefpass-, Bandpass- oder Bandsperrverhalten aufweisen. Die elektrischen Bauteile 25 werden mit der Innenseite der Kontaktkappen 33 bzw. 34 elektrisch verbunden. Wie in Fig. 18 angedeutet ist, erfolgt dies über entsprechende Anschlussleitungen 36, 37, die durch übliche Drähte, Litzen oder Drahtseile gebildet und in üblicher Weise innen an die Kontaktkappen 33, 34 angeschweißt, gekrimpt oder gelasert sein können. Auch eine induktiv oder kapazitiv koppelnde Verbindung der Anschlüsse ist denkbar.

Die beschriebene Ausbildung des Hochfrequenz-Filters 11 als Tonnenfilter führt zu einer Verkürzung der Bauform und einer Erhöhung der Sicherheit durch eine Reduzierung von Verbindungsstellen. Beim Einbau in eine Elektrodenvorrichtung verkürzt sich damit auch der durch den Filter versteifte Bereich, wodurch sich bessere Eigenschaften der Elektrodenvorrichtung in vivo vor allem in Bezug auf die Implantierbarkeit, die Perforationsgefahr und die Langzeitstabilität ergeben.

Wie aus Fig. 19 hervorgeht, kann die elektrische Kontaktierung der Bauteile 25 auch über Schleifkontakte 38 oder entsprechende Kontaktfedern realisiert werden, die mit der Innenseite der Kontaktkappen 33 bzw. 34 elektrisch in Kontakt stehen.

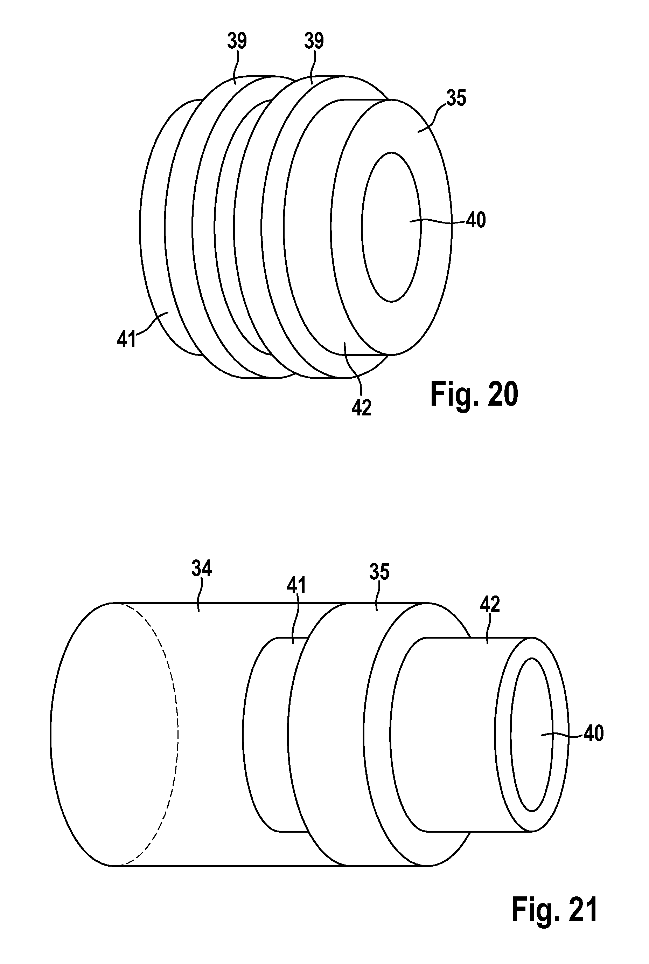

Fig. 20 zeigt eine spezielle Ausbildung des Isolatoreinsatzes 35, der zum einen mit aufgesetzten Schweißscheiben 39 aus Metall versehen ist. Diese stehen radial über die Mantelwand des zylindrisch ausgebildeten Isolatoreinsatzes 35 hinaus und dienen zur Anbindung der Kontaktkappen 33, 34 durch Anschweißen. Ferner weist der Isolatoreinsatz 35 ähnlich einem Rohr koaxial in der Mitte einen Durchgang 40 in Form einer Bohrung oder dergleichen als Aussparung für die Bauteile 25 auf.

Der Isolatoreinsatz 35 kann als isolierendes Zwischenstück z.B. aus Keramik oder Kunststoff bestehen, auf dessen entsprechende Absätze 41, 42 die Kontaktkappen ― in Fig. 21 ist die linke Kontaktkappe 34 gezeigt - aufgeschoben und durch Schweißen, Löten, Kleben, Krimpen oder dergleichen am Isolatoreinsatz 35 befestigt werden können.

Anstatt aus Metall können die zwei Halbtonnen der Kontaktkappen 33, 34 auch aus einem Kunststoff, einem leitfähigen Kunststoff, einer Keramik oder einem anderen Nichtleiter gefertigt werden. Diese müssen dann ganz oder teilweise mit einem leitfähigen Material überzogen sein.

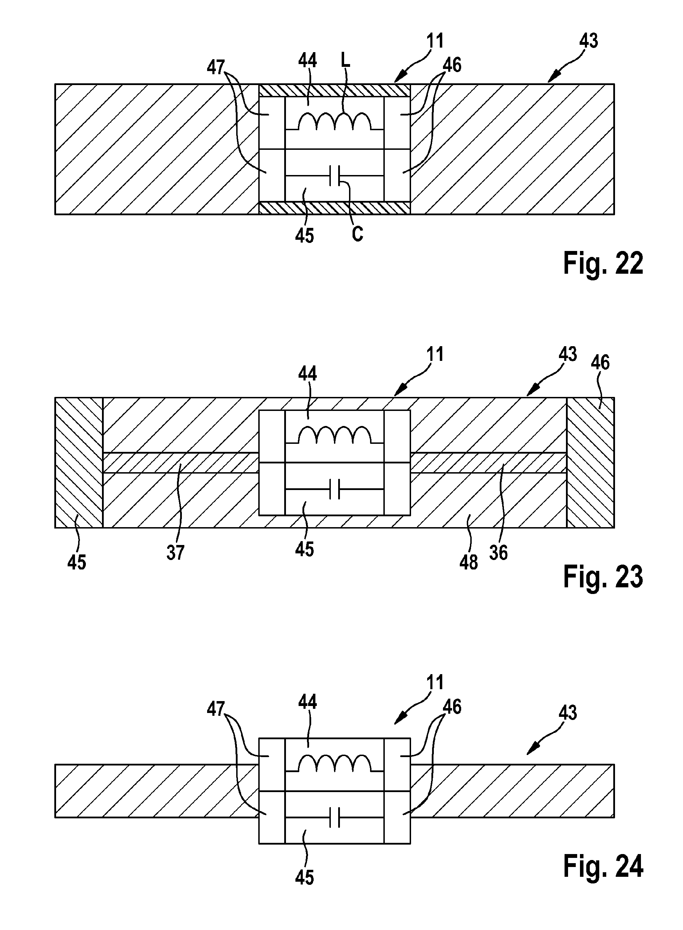

Die Fig. 22 bis 31 schließlich zeigen die integrale Bauweise eines Hochfrequenz-Filters 11 unter direkter Einbettung in einen Spitzen-Kontaktpol 6. Bei einer Integration des Filters 11 für die Tipp-Zuleitung der Elektrodenvorrichtung ist der Filter beispielsweise zwischen der Fixierhelix und der Innenzuleitung des Spitzen-Kontaktpols in einer sogenannten Pin-Einheit 43 untergebracht. Die Integration der Spule und des Filters kann dabei durch diskrete Komponenten, wie beispielsweise SMD-Bauteile erfolgen. Eine Integration durch andere Verfahren ist ebenfalls möglich. Dabei können die Verbindungen zwischen den Elektrodenzuleitungen und dem Filter selbst aus verschiedenen Materialien bestehen, z.B. aus metallischen Werkstoffen, metallischen Werkstoffen mit teilweise isolierender Beschichtung, nicht leitenden Werkstoffen mit integrierten, aufgetragenen elektrischen Leitungen oder geeigneten Kombinationen davon. Mit anderen Worten die Funktion wird statt durch z.B. SMD Komponenten durch gezielte Aneinanderfügung von Materialien mit entsprechenden Eigenschaften erzielt. Z.B. ein Sandwich aus: leitenden Werkstoff, nicht leitenden (besonders dielektrisch ausgeprägter) Werkstoff, leitender Werkstoff bilden in Flussrichtung dieser Aufzählung einen Kondensator. Anderes Beispiel: ein Oxidiertes Metall kann an der Grenzschicht Metall/Metalloxid halbleitende Eigenschaften (Diode) hervorbringen.

Im Folgenden werden verschiedene konkrete Integrationsmöglichkeiten erläutert. So zeigt Fig. 22 ein Bauelement als Pin-Einheit 43, bei dem ein Filter bestehend aus zwei SMD-Bauteilen 44, 45 in Form einer Induktivität L und einer Kapazität C unter deren Parallelverschaltung realisiert ist. Die Bauform der SMD-Bauteile 44, 45 muss nicht gleich sein. Sie sind komplett in die Pin-Einheit integriert, diese kann damit isodiametrisch hergestellt werden. Die Anschlüsse 46, 47 links und rechts bestehen aus leitfähigem Material.

Wie aus Fig. 23 hervorgeht, kann die Pin-Einheit 43 auch aus einem Körper 48 aus dielektrischem Material bestehen, der entsprechende Anschlussleitungen 36, 37 zwischen den die Filterkomponenten realisierenden SMD-Bauteilen 44, 45 und den Anschlüssen 46, 47 aufweist. Diese Bauform beansprucht die Filterkomponenten weniger, da sie in einem homogenen Material eingebettet sind.

Bei der in Fig. 24 gezeigten Ausführungsform ist der Hochfrequenz-Filter 11 mit seinen SMD-Bauteilen 44, 45 in einen Körper 48 mit einer relativ dünnen Struktur integriert. Die Montage zwischen zwei leitfähigen Elementen ist in dieser Zeichnung weggelassen. Die gesamte Pin-Einheit 43 ist dann nicht zwingend isodiametrisch. Wie anhand von Fig. 23 beschrieben ist, kann diese Ausführungsform auch aus dielektrischem Material mit geeigneten Leitungsstrukturen bestehen.

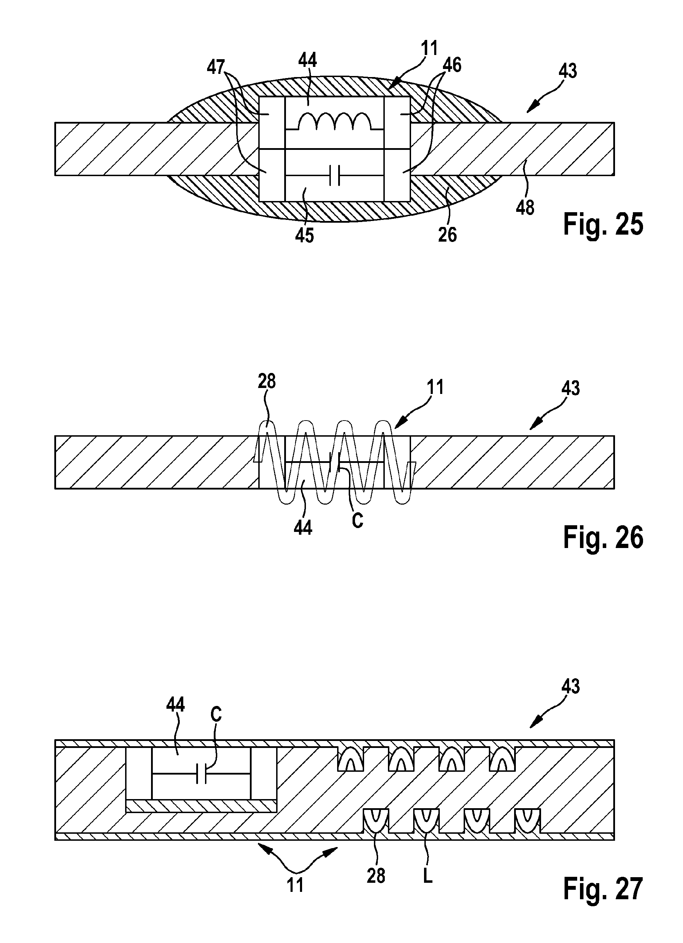

Um den Hochfrequenz-Filter 11 selbst von der Umgebung abzutrennen, wird er - wie Fig. 25 zeigt - mit einem umspritzten Kunststoffkörper 26, einer Beschichtung, einem Gehäuse oder ähnlichen Maßnahmen umhüllt.

Eine weitere Miniaturisierung für die Pin-Einheit 43 wird mit der in Fig. 26 gezeigten Ausführungsform erzielt. Dort ist die Drahtspule 28 des Hochfrequenz-Filters 11 um das als Kapazität C ausgelegte SMD-Bauteil 44 herum angelegt. Damit wird das benötigte Bauvolumen gegenüber den vorher erörterten Ausführungsformen gemäß Fig. 22 bis 25 insbesondere im Durchmesser deutlich verringert.

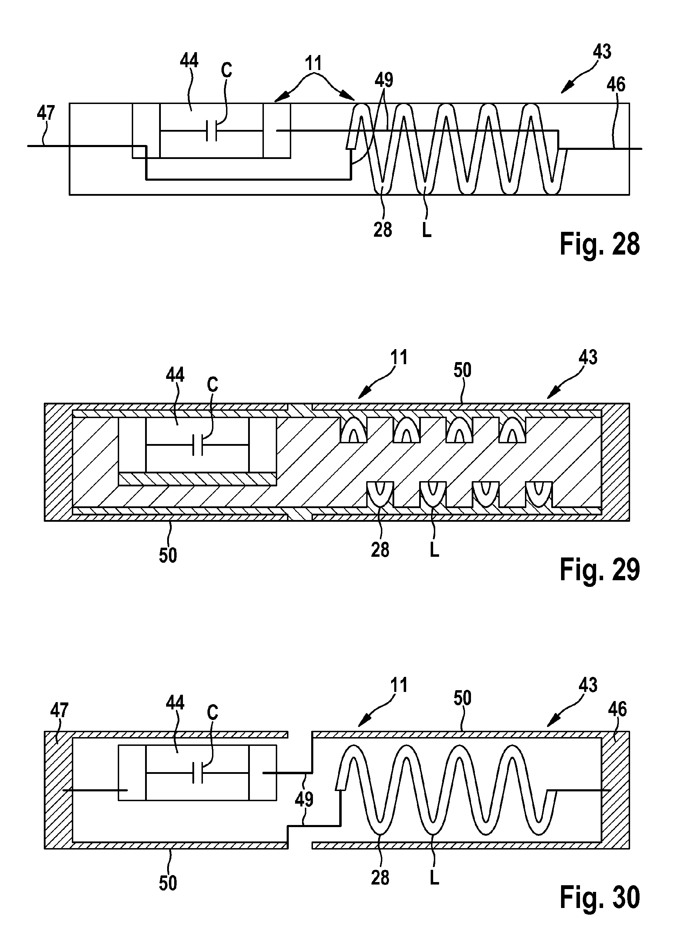

Bei dem in Fig. 28 dargestellten Ausführungsbeispiel der Pin-Einheit 43 werden die Spule 28 und das SMD-Bauteil 44 für die Kapazität C mechanisch hintereinander montiert, wobei die in dicken Linien herausgezeichnete Verschaltung 49 parallel erfolgt. Die Pin-Einheit kann dabei isodiametrisch ausgeführt sein und an ihren Enden die entsprechenden Anschlüsse 46, 47 aufweisen. Die Kapazität C ist als Kondensator-SMD-Bauteil 44 und die Induktivität L als Drahtspule im Inneren des Bauteils montiert. In Fig. 28 ist die tragende Struktur des Körpers der Pin-Einheit der Übersichtlichkeit halber weggelassen.

Eine solche ist in Fig. 29 erkennbar. Ferner weist diese Ausführungsform eine Metallisierung 50 auf, wie sie sich über weite Teilbereiche der Pin-Einheit 43 auf deren Außenseite erstreckt. Über diese Metallisierung 50 erfolgt die Kontaktierung der Bauteile, nämlich der Drahtspule 28 und des Kondensator-SMD-Bauteils 44, wie dies in Fig. 30 erkennbar ist. Die Verschaltung 49 kann damit im Inneren mit kürzeren Wegen auskommen.

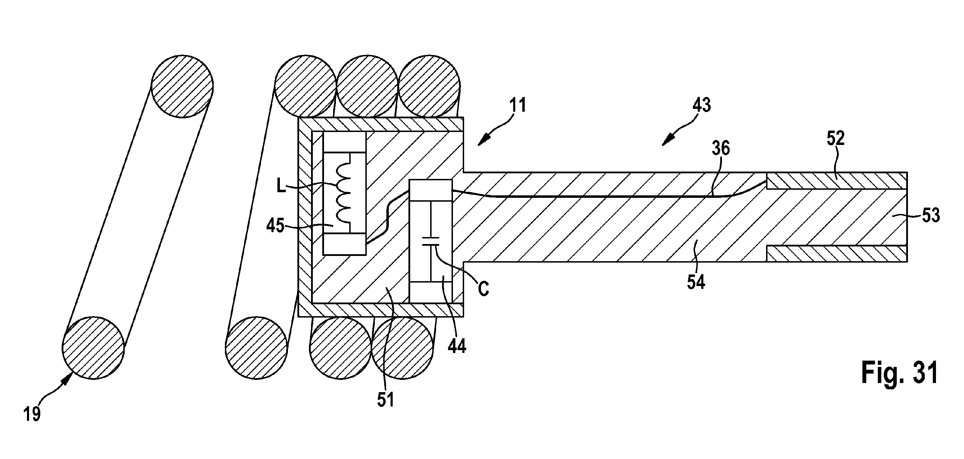

In Fig. 31 schließlich ist eine Ausführungsform gezeigt, bei der die Pin-Einheit 43 an seinem distalen Ende einen größeren Durchmesser besitzt. Auf dem dadurch gebildeten Absatz 51 wird das Schraubteil 19 der so realisierten Fixierhelix 18 des Spitzen-Kontaktpols 6 befestigt. Im Bereich des Absatzes 51 wird der Hochfrequenz-Filter 11 mit seinen SMD-Bauteilen, 44, 45 integriert. Beispielhaft ist in Fig. 31 die Integration eines Bandstopp-Filters mit einem SMD-Bauteil 44 als Kapazität C und einem Bauteil 45 als Induktivität L gezeigt. Der Filter 11 ist über den Anschlussring 52 am proximalen Ende 53 des Schaftes 54 der Pin-Einheit 43 über eine eingebettete Anschlussleitung 36 elektrisch verbunden.

Die vorstehend beschriebenen Konzepte in Verbindung mit den Fig. 22 bis 31 sind mit gewickelten, planaren oder in LTCC integrierten Spulen ebenfalls möglich.

### Bezugszeichenliste

- 1: Distales Ende
- 2: Elektrodenkörper
- 3: Zuleitung außen
- 4: Zuleitung innen
- 5: Ring-Kontaktpol
- 6: Spitzen-Kontaktpol
- 7: Schlauch
- 8: Isolationslage
- 9: Isolationshülse
- 10: Steroidkragen
- 11: Hochfrequenz-Filter
- 12: Gehäuse 12a Außenfläche
- 13: Kontaktpin
- 14: Kontaktpin
- 15: Wendelstück
- 16: Kopf
- 17: Anschluss
- 18: Fixierhelix
- 19: Schraubteil
- 20: Lagerscheibe
- 21: Lagerscheibe
- 22: Lagerbohrung
- 23: Lagerbohrung
- 24: halbkugelförmiger Kopf
- 25: elektrische Bauteile
- 26: Kunststoffkörper
- 27: Beschichtung
- 28: Drahtspule
- 29: Röhrchen
- 30: Röhrchen

- 31: Lumen
- 32: Durchlass
- 33: Kontaktkappe
- 34: Kontaktkappe
- 35: Isolatoreinsatz
- 36: Anschlussleitung
- 37: Anschlussleitung
- 38: Schleifkontakt
- 39: Schweißscheibe
- 40: Duchgang
- 41: Absatz
- 42: Absatz
- 43: Pin-Einheit
- 44: SMD-Bauteil
- 45: SMD-Bauteil
- 46: Anschluss
- 47: Anschluss
- 48: Körper
- 49: Verschaltung
- 50: Metallisierung
- 51: Absatz
- 52: Anschlussring
- 53: proximales Ende (Fig. 31)
- 54: Schaft
- d: Innendurchmesser von 5
- D: Außendurchmesser von 11

## Patentansprüche

1. Elektrodenvorrichtung für aktive medizinische Implantate umfassend
- einen langgestreckten Elektrodenkörper (2) mit einem proximalen und einem distalen Ende (1),
- einen Spitzen-Kontaktpol (6) am distalen Ende (1),
- mindestens einen Ring-Kontaktpol (5) vor dem distalen Ende (1),
- elektrischen Zuleitungen (3, 4) zu dem Spitzen- und Ring-Kontaktpol (6, 5), und
- einen Hochfrequenz-Filter (11) vor dem distalen Ende (1), der elektrischen Kontakt zum Spitzen-Kontaktpol (6) aufweist und in dessen Zuleitung (4) eingeschaltet ist.

2. Elektrodenvorrichtung nach Anspruch 1, **dadurch gekennzeichnet, dass** der Hochfrequenz-Filter 11) zumindest teilweise innerhalb des Ring-Kontaktpols (5) radial überlappend positioniert ist.

3. Elektrodenvorrichtung nach Anspruch 2, **dadurch gekennzeichnet, dass** die vorzugsweise wendelförmige Zuleitung (3) für den Ring-Kontaktpol (5) in proximaler Richtung vor dem Hochfrequenz-Filter (11) endet.

4. Elektrodenvorrichtung nach Anspruch 2, **dadurch gekennzeichnet, das**s die äußere Zuleitung (3) für den Ring-Kontaktpol (5) gleichzeitig mit der Außenfläche (12a) des Gehäuses (12) des Hochfrequenz-Filters (11) in elektrischem Kontakt steht.

5. Elektrodenvorrichtung nach Anspruch 1, **dadurch gekennzeichnet, dass** der Ring-Kontaktpol (5) in proximaler Richtung derart vor dem Hochfrequenz-Filter (11) angeordnet ist, dass sich Ring-Kontaktpol (5) und Hochfrequenz-Filter (11) außerhalb axialer Überdeckung befinden und der Innendurchmesser (d) des Ring-Kontaktpols (5) gegebenenfalls mit der wendelförmigen Zuleitung (3) kleiner als der Außendurchmesser (D) des Hochfrequenz-Filters (11) ist.

6. Elektrodenvorrichtung nach einem der vorgenannten Ansprüche, **dadurch gekennzeichnet, dass** der Hochfrequenz-Filter (11) mit dem Spitzen-Kontaktpol (6) mechanisch und elektrisch durch ein flexibles Wendelstück (15) verbunden ist.

7. Elektrodenvorrichtung nach einem der vorgenannten Ansprüche, **dadurch gekennzeichnet, dass** der Spitzen-Kontaktpol (6) ausgewählt ist aus folgenden Komponenten: ein Kopf (26) mit elektrisch aktiver Fixierschraube (18), ein linsen-, kegel- oder halbkugelförmiger Kopf (24), ein verlängerter, als Fixierschraube (19) ausgebildeter Kontaktpin (14) des Hochfrequenz-Filters (11), oder ein Fortsatz des Gehäuses (12) des Hochfrequenz-Filters (11).

8. Elektrodenvorrichtung nach einem der vorgenannten Ansprüche, **dadurch gekennzeichnet, dass** der Hochfrequenz-Filter (11) gemeinsam mit dem als elektrisch aktive Fixierschraube (18) ausgebildeten Spitzen-Kontaktpol (6) in der Elektrodenvorrichtung, insbesondere innerhalb des Ring-Kontaktpols (5) um die Längsachse der Elektrodenvorrichtung drehbar gelagert ist.

9. Elektrodenvorrichtung nach Anspruch 8, **dadurch gekennzeichnet, dass** der Hochfrequenz-Filter (11) mit seinen Kontaktpins (13, 14) in Lagerbohrungen (22, 23) von Lagerscheiben (20, 21) drehbar gelagert und durch die elektrische Zuleitung (4) des Spitzen-Kontaktpols (6) rotationsangetrieben ist.

10. Elektrodenvorrichtung nach einem der vorgenannten Ansprüche, **dadurch gekennzeichnet, dass** der Hochfrequenz-Filter (11) elektrische Kontaktpins (13, 14) mit dazwischen geschalteten elektronischen Miniatur-Bauelementen (25) aufweist, wobei Kontaktpins (13, 14) und Miniatur-Bauelemente (25) gemeinsam stoffschlüssig in einem umspritzten Filtergehäuse (26) angeordnet sind.

11. Elektrodenvorrichtung nach Anspruch 10, **dadurch gekennzeichnet, dass** die Außenseite des Filtergehäuses (26) mit einer funktionalen Beschichtung (27) versehen ist.

12. Elektrodenvorrichtung nach Anspruch 10 oder 11, **dadurch gekennzeichnet, dass** die Kontaktpins (13, 14) jeweils als Röhrchen (29, 30) ausgebildet sind, deren Lumen (31) mit einem Durchlass (32) im Filtergehäuse (26) als Durchgang für einen Führungsdraht zur Implantierung der Elektrodenvorrichtung fluchten.

13. Elektrodenvorrichtung nach einem der Ansprüche 1 bis 9, **dadurch gekennzeichnet, dass** die Kontaktpins (12, 13) des Hochfrequenz-Filters (11) als einander zugewandte, voneinander isolierte Kontaktkappen (33, 34) ausgebildet sind, in deren Innenraum die elektrischen Komponenten (25) des Hochfrequenz-Filters (11) angeordnet sind.

14. Elektrodenvorrichtung nach Anspruch 13, **dadurch gekennzeichnet, dass** die Anschlüsse (36, 37) der elektrischen Komponenten (25) mit der Innenseite der Kontaktkappen, vorzugsweise über Schleifkontakte (38) oder Kontaktfedern, in elektrischem Kontakt stehen.

15. Elektrodenvorrichtung nach einem der vorgenannten Ansprüche, **dadurch gekennzeichnet, dass** der Hochfrequenz-Filter (11) direkt in den Spitzen-Kontaktpol (6) integriert ist.
